# EUROPEAN PATENT APPLICATION

(11) **EP 0 884 386 A1**
(43) Date of publication of application: **16.12.1998**
(21) Application number: 97401196.7
(22) Date of filing: 30.05.1997
(51) Int. Cl.: C12N 15/12, C07K 14/705, C07K 16/18, C12N 5/10, A61K 38/17, G01N 33/50

(54) **Sodium channel receptor**

(71) Applicant: SYNTHELABO, 92350 Le Plessis Robinson (FR)
(72) Inventor: Renard, Stéphane, 92500 Rueil Malmaison (FR); Besnard, François, 78220 Viroflay (FR); Graham, David, 78380 Bougival (FR)
(74) Representative: Thouret-Lemaitre, Elisabeth

(57) **Abstract**

hSLNAC1 polypeptides and polynucleotides and methods for producing such polypeptides by recombinant techniques are disclosed. Also disclosed are methods for utilizing hSLNAC1 polypeptides and polynucleotides in the design of protocols for the treatment of certain diseases and diagnostic assays for such conditions.

SEQUENCE LISTING

(1) GENERAL INFORMATION:

(i) APPLICANT:
(A) NAME: Synthelabo
(B) STREET: 22, Avenue Galilée
(C) CITY:Le Plessis Robinson
(E) COUNTRY: France
(F) POSTAL CODE (ZIP): 92350

(ii) TITLE OF INVENTION: Sodium channel receptor

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS/Windows 3.1
(D) SOFTWARE: WordPerfect Version 6.1

(v) CURRENT APPLICATION DATA:
APPLICATION NUMBER: EP 9205558

(2) INFORMATION FOR SEQ ID NO: 1:

(i) SEQUENCE CHARACTERISTICS:
(A) LENGTH: 1711 base pairs
(B) TYPE: nucleic acid
(C) STRANDEDNESS: double
(D) TOPOLOGY: circular

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Homo sapiens
(F) TISSUE TYPE: Cerebellum

(vii) IMMEDIATE SOURCE:
(B) CLONE: SLNAC1

(ix) FEATURE:
(A) NAME/KEY: CDS
(B) LOCATION:76..1632
(D) OTHER INFORMATION:/codon_start= 76
/function= "sodium channel receptor"
/product= "SLNAC1"
/standard_name= "slnac1"
/label= slnac1

(ix) FEATURE:
(A) NAME/KEY: mat_peptide
(B) LOCATION:76..1629

(D) OTHER INFORMATION:/codon_start= 76
/function= "sodium channel receptor"
/product= "h_SLNAC1"

(ix) FEATURE:
(A) NAME/KEY: misc_feature
(B) LOCATION:1..75
(D) OTHER INFORMATION:/product= "plasmidic polylinker"

(ix) FEATURE:
(A) NAME/KEY: misc_feature
(B) LOCATION:1630..1711

(xi) SEQUENCE DESCRIPTION: SEQ ID NO: 1:

CTGGAGCTCC ACCGCGGTGG CGGCCGCTCT AGAACTAGTG GATCCCCCGG GCTGCAGGAA 60

TTCGGCACGA GCTCG ATG CAC GGG CTG GGC CAC GTC TTC GGG CCA GGC AGC 111
Met His Gly Leu Gly His Val Phe Gly Pro Gly Ser
1               5                   10

CTG AGC CTG CGC CGG GGG ATG TGG GCA GCG GCC GTG GTC CTG TCA GTG 159
Leu Ser Leu Arg Arg Gly Met Trp Ala Ala Ala Val Val Leu Ser Val
        15                  20                  25

GCC ACC TTC CTC TAC CAG GTG GCT GAG AGG GTG CGC TAC TAC AGG GAG 207
Ala Thr Phe Leu Tyr Gln Val Ala Glu Arg Val Arg Tyr Tyr Arg Glu
    30                  35                  40

TTC CAC CAC CAG ACT GCC CTG GAT GAG CGA GAA AGC CAC CGG CTC ATC 255
Phe His His Gln Thr Ala Leu Asp Glu Arg Glu Ser His Arg Leu Ile
45                  50                  55                  60

TTC CCG GCT GTC ACC CTG TGC AAC ATC AAC CCA CTG CGC CGC TCG CGC 303
Phe Pro Ala Val Thr Leu Cys Asn Ile Asn Pro Leu Arg Arg Ser Arg
                65                  70                  75

CTA ACG CCC AAC GAC CTG CAC TGG GCT GGG TCT GCG CTG CTG GGC CTG 351
Leu Thr Pro Asn Asp Leu His Trp Ala Gly Ser Ala Leu Leu Gly Leu
            80                  85                  90

GAT CCC GCA GAG CAC GCC GCC TTC CTG CGC GCC CTG GGC CGG CCC CCT 399
Asp Pro Ala Glu His Ala Ala Phe Leu Arg Ala Leu Gly Arg Pro Pro
        95                  100                 105

GCA CCG CCC GGC TTC ATG CCC AGT CCC ACC TTT GAC ATG GCG CAA CTC 447
Ala Pro Pro Gly Phe Met Pro Ser Pro Thr Phe Asp Met Ala Gln Leu
    110                 115                 120

TAT GCC CGT GCT GGG CAC TCC CTG GAT GAC ATG CTG CTG GAC TGT CGC 495
Tyr Ala Arg Ala Gly His Ser Leu Asp Asp Met Leu Leu Asp Cys Arg
125                 130                 135                 140

TTC CGT GGC CAA CCT TGT GGG CCT GAG AAC TTC ACC ACG ATC TTC ACC 543
Phe Arg Gly Gln Pro Cys Gly Pro Glu Asn Phe Thr Thr Ile Phe Thr
                145                 150                 155

CGG ATG GGA AAG TGC TAC ACA TTT AAC TCT GGC GCT GAT GGG GCA GAG 591
Arg Met Gly Lys Cys Tyr Thr Phe Asn Ser Gly Ala Asp Gly Ala Glu
            160                 165                 170

CTG CTC ACC ACT ACT AGG GGT GGC ATG GGC AAT GGG CTG GAC ATC ATG 639
Leu Leu Thr Thr Thr Arg Gly Gly Met Gly Asn Gly Leu Asp Ile Met
        175                 180                 185

CTG GAC GTG CAG CAG GAG GAA TAT CTA CCT GTG TGG AGG GAC AAT GAG 687
Leu Asp Val Gln Gln Glu Glu Tyr Leu Pro Val Trp Arg Asp Asn Glu
    190                 195                 200

GAG ACC CCG TTT GAG GTG GGG ATC CGA GTG CAG ATC CAC AGC CAG GAG 735
Glu Thr Pro Phe Glu Val Gly Ile Arg Val Gln Ile His Ser Gln Glu
205                 210                 215                 220

GAG CCG CCC ATC ATC GAT CAG CTG GGC TTG GGG GTG TCC CCG GGC TAC 783
Glu Pro Pro Ile Ile Asp Gln Leu Gly Leu Gly Val Ser Pro Gly Tyr
                225                 230                 235

CAG ACC TTT GTT TCT TGC CAG CAG CAG CAG CTG AGC TTC CTG CCA CCG 831
Gln Thr Phe Val Ser Cys Gln Gln Gln Gln Leu Ser Phe Leu Pro Pro
            240                 245                 250

CCC TGG GGC GAT TGC AGT TCA GCA TCT CTG AAC CCC AAC TAT GAG CCA 879
Pro Trp Gly Asp Cys Ser Ser Ala Ser Leu Asn Pro Asn Tyr Glu Pro
        255                 260                 265

GAG CCC TCT GAT CCC CTA GGC TCC CCC AGC CCC AGC CCC AGC CCT CCC 927
Glu Pro Ser Asp Pro Leu Gly Ser Pro Ser Pro Ser Pro Ser Pro Pro
    270                 275                 280

TAT ACC CTT ATG GGG TGT CGC CTG GCC TGC GAA ACC CGC TAC GTG GCT 975
Tyr Thr Leu Met Gly Cys Arg Leu Ala Cys Glu Thr Arg Tyr Val Ala
285                 290                 295                 300

CGG AAG TGC GGC TGC CGA ATG GTG TAC ATG CCA GGC GAC GTG CCA GTG 1023
Arg Lys Cys Gly Cys Arg Met Val Tyr Met Pro Gly Asp Val Pro Val
                305                 310                 315

TGC AGC CCC CAG CAG TAC AAG AAC TGT GCC CAC CCG GCC ATA GAT GCC 1071
Cys Ser Pro Gln Gln Tyr Lys Asn Cys Ala His Pro Ala Ile Asp Ala
            320                 325                 330

ATG CTT CGC AAG GAC TCG TGC GCC TGC CCC AAC CCG TGC GCC AGC ACG 1119
Met Leu Arg Lys Asp Ser Cys Ala Cys Pro Asn Pro Cys Ala Ser Thr
        335                 340                 345

CGC TAC GCC AAG GAG CTC TCC ATG GTG CGG ATC CCG AGC CGC GCC GCC 1167
Arg Tyr Ala Lys Glu Leu Ser Met Val Arg Ile Pro Ser Arg Ala Ala
    350                 355                 360

GCG CGC TTC CTG GCC CGG AAG CTC AAC CGC AGC GAG GCC TAC ATC GCG 1215
Ala Arg Phe Leu Ala Arg Lys Leu Asn Arg Ser Glu Ala Tyr Ile Ala
365                 370                 375                 380

GAG AAC GTG CTG GCC CTG GAC ATC TTC TTT GAG GCC CTC AAC TAT GAG     1263
Glu Asn Val Leu Ala Leu Asp Ile Phe Phe Glu Ala Leu Asn Tyr Glu
                385                 390                 395

ACC GTG GAG CAG AAG AAG GCC TAT GAG ATG TCA GAG CTG CTT GGT GAC     1311
Thr Val Glu Gln Lys Lys Ala Tyr Glu Met Ser Glu Leu Leu Gly Asp
            400                 405                 410

ATT GGG GGC CAG ATG GGG CTG TTC ATC GGG GCC AGC CTG CTC ACC ATC     1359
Ile Gly Gly Gln Met Gly Leu Phe Ile Gly Ala Ser Leu Leu Thr Ile
        415                 420                 425

CTC GAG ATC CTA GAC TAC CTC TGT GAG GTG TTC CGA GAC AAG GTC CTG     1407
Leu Glu Ile Leu Asp Tyr Leu Cys Glu Val Phe Arg Asp Lys Val Leu
    430                 435                 440

GGA TAT TTC TGG AAC CGA CAG CAC TCC CAA AGG CAC TCC AGC ACC AAT     1455
Gly Tyr Phe Trp Asn Arg Gln His Ser Gln Arg His Ser Ser Thr Asn
445                 450                 455                 460

CTG ACC TCC CAC CCC TCC CTG TGC CGT CAC CAA GAC TCT CTC CGC CTC     1503
Leu Thr Ser His Pro Ser Leu Cys Arg His Gln Asp Ser Leu Arg Leu
                465                 470                 475

CCA CCG CAC CTG CTA CCT TGT CAC ACA GCT CTA GAC CTG CTG TCT GTG     1551
Pro Pro His Leu Leu Pro Cys His Thr Ala Leu Asp Leu Leu Ser Val
            480                 485                 490

TCC TCG GAG CCC CGC CCT GAC ATC CTG GAC ATG CCT AGC CTG CAC GTA     1599
Ser Ser Glu Pro Arg Pro Asp Ile Leu Asp Met Pro Ser Leu His Val
        495                 500                 505

GCT TTT CCG TCT TCA CCC CAA ATA AAG TCC TAA TGCATCAAAA AAAAAAAAAA     1652
Ala Phe Pro Ser Ser Pro Gln Ile Lys Ser  *
    510                 515

AAAAAAACTC GAGGGGGGGC CCGGTACCCA ATTCGCCCTA TAGTGAGTCG TATTACAAT     1711

(2) INFORMATION FOR SEQ ID NO: 2:

(i) SEQUENCE CHARACTERISTICS:
(A) LENGTH: 519 amino acids
(B) TYPE: amino acid
(D) TOPOLOGY: linear

(ii) MOLECULE TYPE: protein
(xi) SEQUENCE DESCRIPTION: SEQ ID NO: 2:

Met His Gly Leu Gly His Val Phe Gly Pro Gly Ser Leu Ser Leu Arg
  1               5                  10                  15

Arg Gly Met Trp Ala Ala Ala Val Val Leu Ser Val Ala Thr Phe Leu
             20                  25                  30

Tyr Gln Val Ala Glu Arg Val Arg Tyr Tyr Arg Glu Phe His His Gln
         35                  40                  45

Thr Ala Leu Asp Glu Arg Glu Ser His Arg Leu Ile Phe Pro Ala Val
    50                  55                  60

Thr Leu Cys Asn Ile Asn Pro Leu Arg Arg Ser Arg Leu Thr Pro Asn
65                  70                  75                  80

Asp Leu His Trp Ala Gly Ser Ala Leu Leu Gly Leu Asp Pro Ala Glu
                85                  90                  95

His Ala Ala Phe Leu Arg Ala Leu Gly Arg Pro Pro Ala Pro Pro Gly
            100                 105                 110

Phe Met Pro Ser Pro Thr Phe Asp Met Ala Gln Leu Tyr Ala Arg Ala
        115                 120                 125

Gly His Ser Leu Asp Asp Met Leu Leu Asp Cys Arg Phe Arg Gly Gln
    130                 135                 140

Pro Cys Gly Pro Glu Asn Phe Thr Thr Ile Phe Thr Arg Met Gly Lys
145                 150                 155                 160

Cys Tyr Thr Phe Asn Ser Gly Ala Asp Gly Ala Glu Leu Leu Thr Thr
                165                 170                 175

Thr Arg Gly Gly Met Gly Asn Gly Leu Asp Ile Met Leu Asp Val Gln
            180                 185                 190

Gln Glu Glu Tyr Leu Pro Val Trp Arg Asp Asn Glu Glu Thr Pro Phe
        195                 200                 205

Glu Val Gly Ile Arg Val Gln Ile His Ser Gln Glu Glu Pro Pro Ile
    210                 215                 220

Ile Asp Gln Leu Gly Leu Gly Val Ser Pro Gly Tyr Gln Thr Phe Val
225                 230                 235                 240

Ser Cys Gln Gln Gln Gln Leu Ser Phe Leu Pro Pro Pro Trp Gly Asp
                245                 250                 255

Cys Ser Ser Ala Ser Leu Asn Pro Asn Tyr Glu Pro Glu Pro Ser Asp
            260                 265                 270

Pro Leu Gly Ser Pro Ser Pro Ser Pro Ser Pro Pro Tyr Thr Leu Met
        275                 280                 285

Gly Cys Arg Leu Ala Cys Glu Thr Arg Tyr Val Ala Arg Lys Cys Gly
    290                 295                 300

Cys Arg Met Val Tyr Met Pro Gly Asp Val Pro Val Cys Ser Pro Gln
305                 310                 315                 320

Gln Tyr Lys Asn Cys Ala His Pro Ala Ile Asp Ala Met Leu Arg Lys
                325                 330                 335

Asp Ser Cys Ala Cys Pro Asn Pro Cys Ala Ser Thr Arg Tyr Ala Lys
            340                 345                 350

Glu Leu Ser Met Val Arg Ile Pro Ser Arg Ala Ala Ala Arg Phe Leu
        355                 360                 365

Ala Arg Lys Leu Asn Arg Ser Glu Ala Tyr Ile Ala Glu Asn Val Leu
    370                 375                 380

Ala Leu Asp Ile Phe Phe Glu Ala Leu Asn Tyr Glu Thr Val Glu Gln
385                 390                 395                 400

Lys Lys Ala Tyr Glu Met Ser Glu Leu Leu Gly Asp Ile Gly Gly Gln
                405                 410                 415

Met Gly Leu Phe Ile Gly Ala Ser Leu Leu Thr Ile Leu Glu Ile Leu
            420                 425                 430

Asp Tyr Leu Cys Glu Val Phe Arg Asp Lys Val Leu Gly Tyr Phe Trp
        435                 440                 445

Asn Arg Gln His Ser Gln Arg His Ser Ser Thr Asn Leu Thr Ser His
    450                 455                 460

Pro Ser Leu Cys Arg His Gln Asp Ser Leu Arg Leu Pro Pro His Leu
465                 470                 475                 480

Leu Pro Cys His Thr Ala Leu Asp Leu Leu Ser Val Ser Ser Glu Pro
                485                 490                 495

Arg Pro Asp Ile Leu Asp Met Pro Ser Leu His Val Ala Phe Pro Ser
            500                 505                 510

Ser Pro Gln Ile Lys Ser *
        515

## Description

### FIELD OF INVENTION

This invention relates to newly identified polynucleotides, polypeptides encoded by them and to the use of such polynucleotides and polypeptides, and to their production. More particularly, the polypeptides of the present invention is a sodium channel receptor, hereinafter referred to as hSLNAC1.

### BACKGROUND OF THE INVENTION

The mdeg and epithelial sodium channel family is a recent family of proteins composed of the homomeric or multimeric assembly of two transmembrane domain polypeptides to form a sodium channel (Renard S. et al (1994) J. Biol. Chem. 269/17 pp12981-12986 ; Lingueglia E.et al (1994) J. Biol. Chem. 269/19, 13736-13739 ; Renard S., et al (1995) Pflügers Archiv- Eur. J. Physiol 430,299-307 ; Lingueglia et al (1995) Nature 378, 730-733 ; Waldmann et al (1996) J. Biol. Chem. 271, 10433-10436). Members of this family have been described as sodium channels or putative mecanosensitive channels, in numerous tissues from nematode to man. The presence of a large extracellular domain in this protein class suggests that they may play the role of a receptor for some endogenous transmitters. Opening of the channel may be linked to this receptor function. Pharmacological properties of such a receptor are still largely unknown, but the diuretic amiloride is known to block most sodium channels of this family. Agonists or antagonists may be used, for example, to treat neuronal degenerescence problems, hyperalgesia, Alzeihmer disease, Parkinson disease, chorea, muscular spasm, epilepsy, stroke, cardiac diseases, schizophrenia, depression, nicotine dependence, morphine dependence, amyotrophic lateral sclerosis, multiple sclerosis, inflammation, pain, cancer, obesity, to mimic or antagonize effect of some endogenous transmitter peptides in the central or peripheral nervous system, such as for instance opioïds or anti-opioïds, to alter gustative perception, to cause analgesia or anesthesia, or to diagnose or treat any disorder related to abnormal expression of this protein.

### SUMMARY OF THE INVENTION

The present inventors have found a new class of sodium channel protein. This new subunit may be responsible for some nervous system transmissions, disorders or may be a target to regulate some transmissions linked to various pathologies.
In accordance with one aspect of the present invention, there is provided a novel mature polypeptide which is a sodium channel, as well as fragments, analogs or derivatives. The polypeptide of the present invention is of human origin.
In accordance with another aspect of the present invention, there are provided polynucleotides which encode such polypeptide.

In accordance with yet a further aspect of the present invention, there is provided a process for producing such polypeptide by recombinant techniques.

In still another aspect, the invention relates to methods to identify agonists and antagonists using the materials provided by the invention, and treating conditions associated with hSLNAC1 imbalance with said identified compounds.

Yet another aspect of the invention relates to diagnostic assays for detecting diseases associated with inappropriate hSLNAC1 activity or levels.

These and other aspects of the present invention should be apparent to those skilled in the art from the teachings herein.

### Brief Description of the Figures

The following drawings are illustrative of the embodiments of the invention and are not meant to limit the scope of the invention as encompassed by the claims.

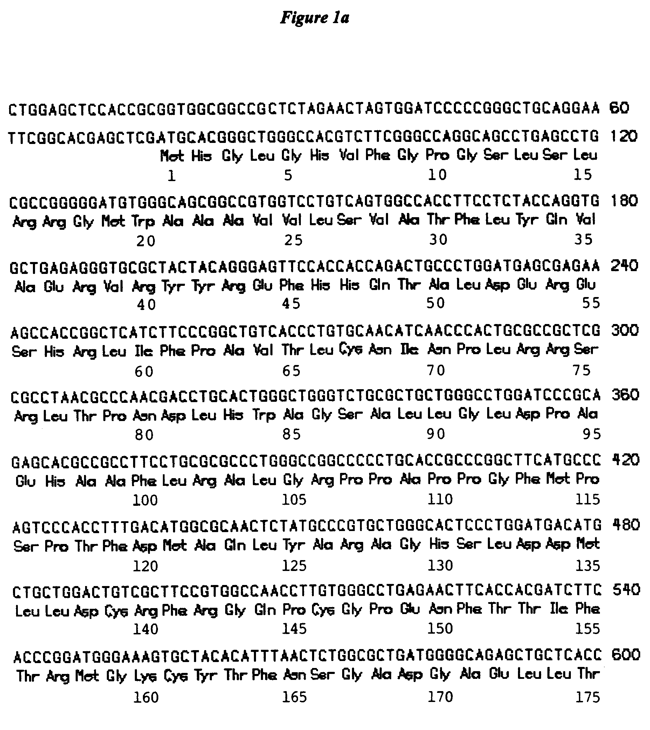

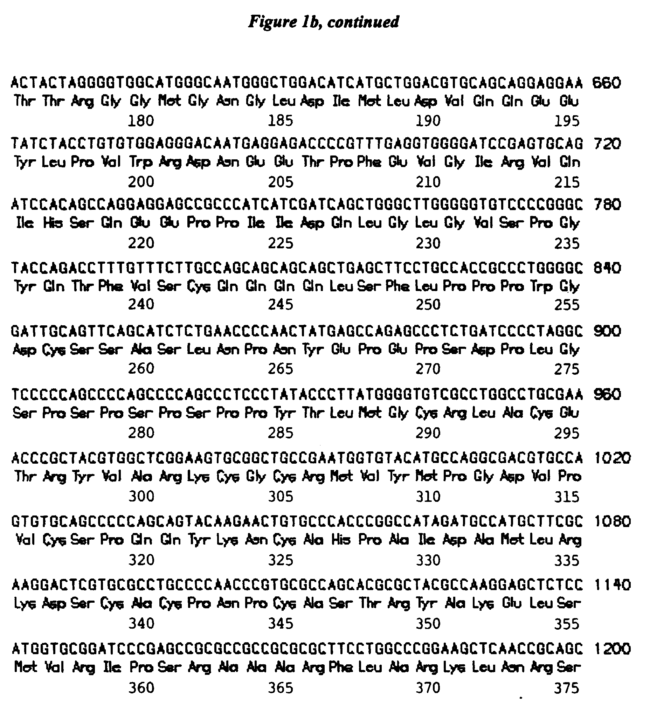

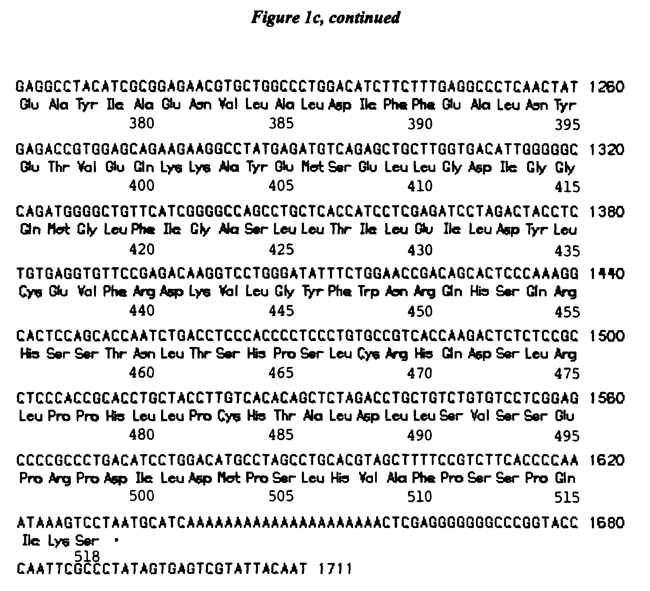

***Figures 1a**, **1b and 1c***, show the cDNA SEQ ID NO:1 from 5' to 3' and the corresponding deduced amino-acid sequence of hSLNAC1 receptor : SEQ ID NO:2. The open reading frame of cDNA SEQ ID NO:1 begins in 76 and ends in 1629. The full length protein SEQ ID NO:2 is 518 amino acid residues with a putative molecular weight of 57656 Daltons.

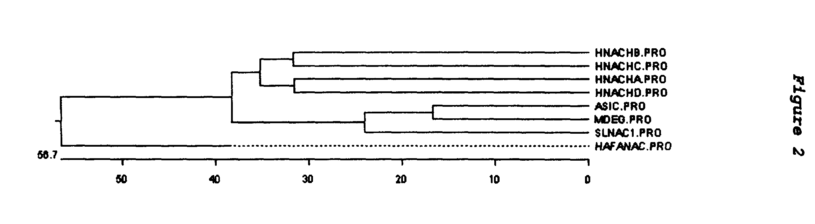

***Figure 2*** illustrates the phylogenic relationship between hSLNAC1 and other members of this sodium channel family. The length of each pair of branches represents the distance between sequence pairs. Alignment was performed with the clustal algorythm of MEGALIGN (version 3.10a ) from DNASTAR.

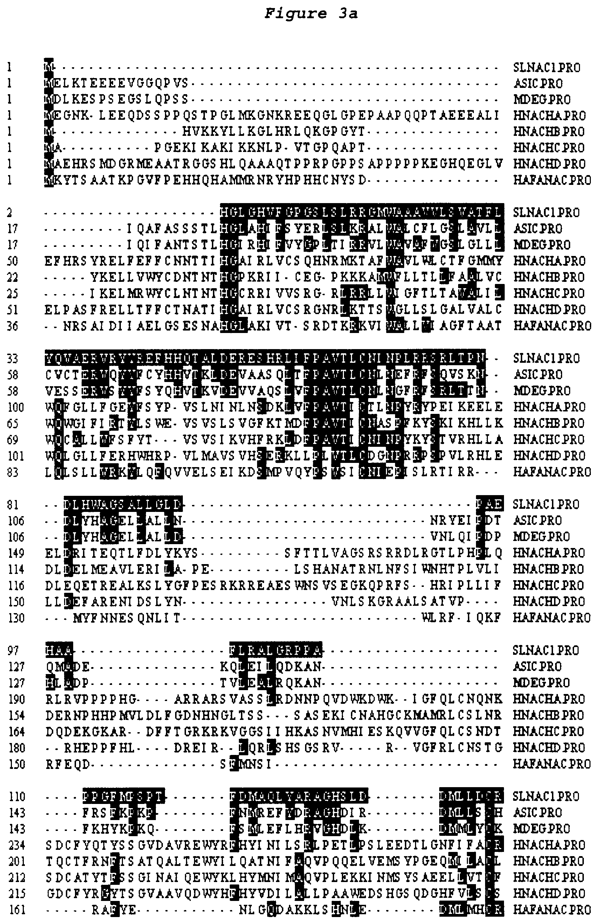

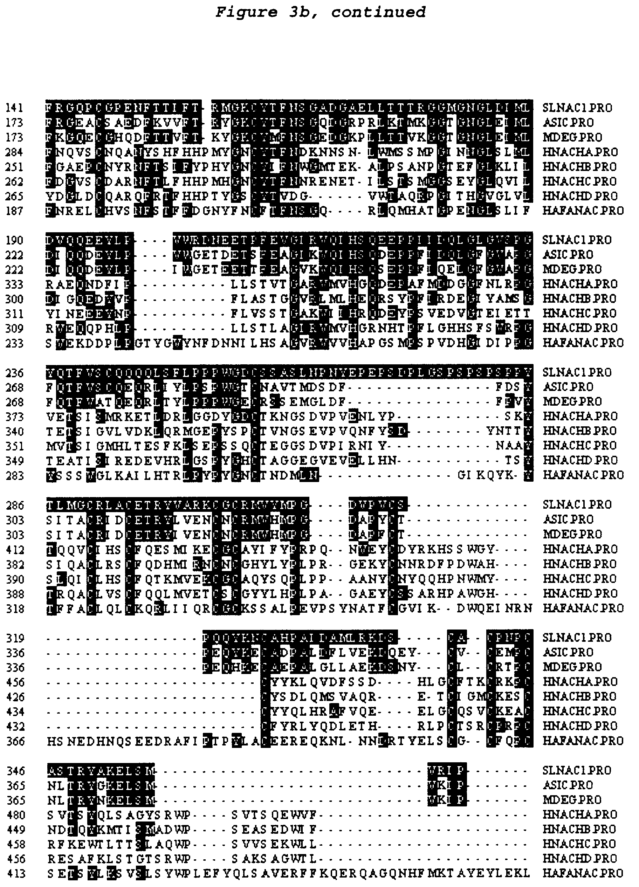

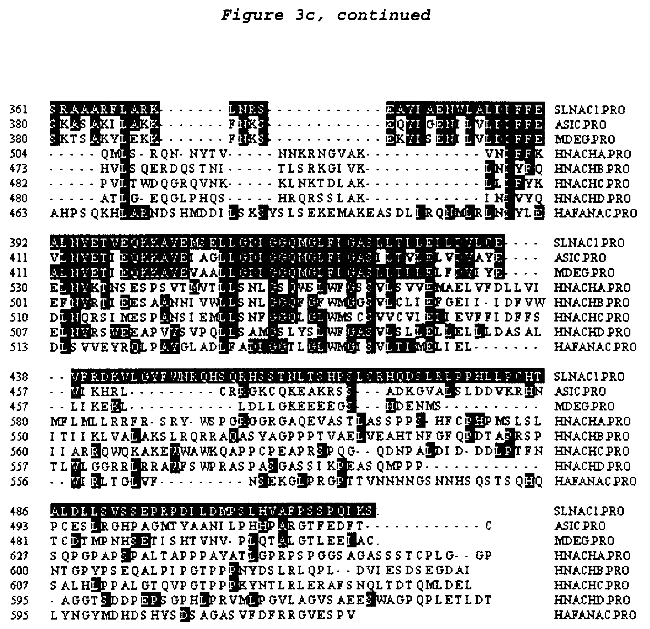

***Figure 3*** shows an aminoacid sequence comparison of the hSLNAC1 receptor with other members of the sodium channel family from the phylogenic tree of Figure 2. The alignment was performed with the clustal algorythm of MEGALIGN(version 3.10a ) from DNASTAR. Regions of homology to SLNAC1 are shaded in black.
   In Figures 2 and 3, the terms used have the following meanings :
   hNACHA : alpha subunit of epithelial sodium channel (accession SW SCAA_HUMAN)
   hNACHB : beta subunit of epithelial sodium channel (accession PIR I38203)
   hNACHC : gamma subunit of epithelial sodium channel (accession PIR I38204)
   hNACHD : delta subunit of sodium channel (accession PIR I39196)
   ASIC : ASIC sodium channel (as published in Nature 368, 173-177- accession RNU94403)
   MDEG : MDEG sodium channel (accession gi 1280439)
   HAFANACH : sodium channel protein from aplysia, gated by FRMF-amide (accession gi 1149511).

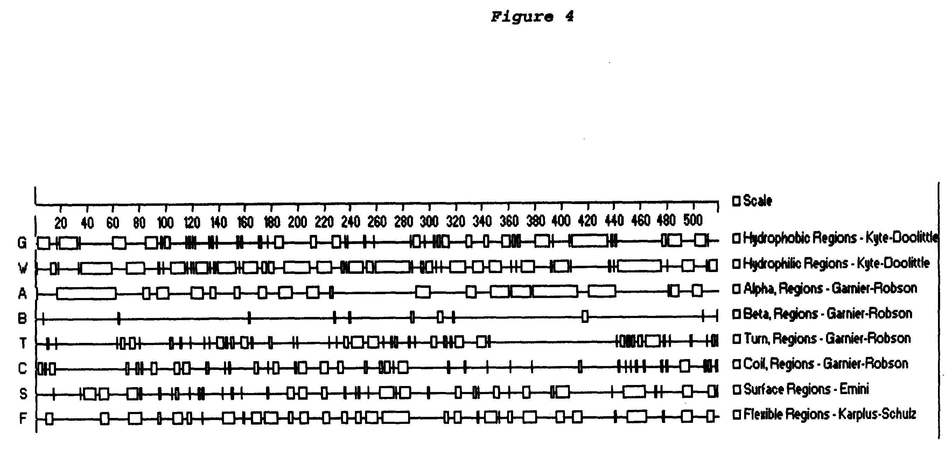

***Figure 4*** illustrates secondary structural features of this hSLNAC1 protein with the hydrophilicity, hydrophobicity, the propency to generate alpha helix, beta sheet, turn or coiled regions, the propency to be on surface of the protein, and the flexible regions. The boxed areas are the areas which correspond to the regions indicated. The hydrophobicity plot illustrates hydrophobic areas of the protein sequence which are in the lipid bilayer and hydrophilic areas which are outside the lipid bilayer. The antigenicity of the protein fragments is higher in areas exposed to the surface, which are hydrophilic and flexible regions. The analysis was performed with Protean (version 3.08a) from DNASTAR.

### DESCRIPTION OF THE INVENTION

### Definitions

The following definitions are provided to facilitate understanding of certain terms used frequently herein.

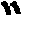 Receptor Activity*"* or 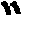 Biological Activity of the Receptor*"* refers to the metabolic or physiologic function of said hSLNAC1 including similar activities or improved activities or these activities with decreased undesirable side-effects. Also included are antigenic and immunogenic activities of said hSLNAC1.
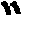 hSLNAC1 polypeptide*"* refers among others to a polypeptide comprising the amino acid sequence set forth in SEQ ID NO:2 or an allelic variant thereof.
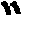 hSLANC1 gene*"* or 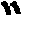 hSLNAC1 polynucleotide*"* refer to a polynucleotide comprising the nucleotide sequence set forth in SEQ ID NO:1 or allelic variants thereof and/or their complements.

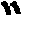 Antibodies*"* as used herein includes polyclonal and monoclonal antibodies, chimeric, single chain, and humanized antibodies, as well as Fab fragments, including the products of an Fab or other immunoglobulin expression library.

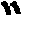 Isolated*"* means altered 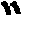 by the hand of man*"* from the natural state. If an 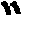 isolated*"* composition or substance occurs in nature, it has been changed or removed from its original environment, or both. For example, a polynucleotide or a polypeptide naturally present in a living animal is not 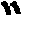 isolated,*"* but the same polynucleotide or polypeptide separated from the coexisting materials of its natural state is 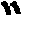 isolated*"*, as the term is employed herein.

"Polynucleotide" generally refers to any polyribonucleotide or polydeoxyribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, 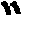 polynucleotide*"* refers to triple-stranded regions comprising RNA or DNA or both RNA and DNA. The term polynucleotide also includes DNAs or RNAs containing one or more modified bases and DNAs or RNAs with backbones modified for stability or for other reasons. 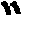 Modified*"* bases include, for example, tritylated bases and unusual bases such as inosine. A variety of modifications has been made to DNA and RNA; thus, 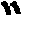 polynucleotide*"* embraces chemically, enzymatically or metabolically modified forms of polynucleotides as typically found in nature, as well as the chemical forms of DNA and RNA characteristic of viruses and cells. 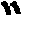 Polynucleotide*"* also embraces relatively short polynucleotides, often referred to as oligonucleotides.

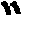 Polypeptide*"* refers to any peptide or protein comprising two or more amino acids joined to each other by peptide bonds or modified peptide bonds, i.e., peptide isosteres. 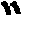 Polypeptide*"* refers to both short chains, commonly referred to as peptides, oligopeptides or oligomers, and to longer chains, generally referred to as proteins. Polypeptides may contain amino acids other than the 20 gene-encoded amino acids. 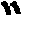 Polypeptides*"* include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al*., 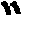 Analysis for protein modifications and nonprotein cofactors*"*, *Meth Enzymol* (1990) 182:626-646 and Rattan *et al*., 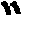 Protein Synthesis: Posttranslational Modifications and Aging*"*, *Ann NY Acad Sci* (1992) 663:48-62.

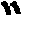 Variant*"* as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

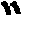 Identity*"* is a measure of the identity of nucleotide sequences or amino acid sequences. In general, the sequences are aligned so that the highest order match is obtained. 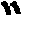 Identity*" per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al*., *Nucleic Acids Research* (1984) 12(1):387), BLASTP, BLASTN, FASTA (Atschul, S.F. *et al*., *J Molec Biol* (1990) 215:403).

As an illustration, by a polynucleotide having a nucleotide sequence having at least, for example, 95% "identity" to a reference nucleotide sequence of SEQ ID NO : 1 is intended that the nucleotide sequence of the polynucleotide is identical to the reference sequence except that the polynucleotide sequence may include up to five point mutations per each 100 nucleotides of the reference nucleotide sequence of SEQ ID NO : 1 In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of Figure 3 is intended that the amino acid sequence of the polypeptide is identical to the reference sequence except that the polypeptide sequence may include up to five amino acid alterations per each 100 amino acids of the reference amino acid of SEQ ID NO : 1. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to hSLNAC1 polypeptides. The hSLNAC1 polypeptides include the polypeptide of SEQ ID NO:2; as well as polypeptides comprising the amino acid sequence of SEQ ID NO:2; and polypeptides comprising the amino acid sequence which have at least 80% identity to that of SEQ ID NO:2 over its entire length, and still more preferably at least 90% identity, and even still more preferably at least 95% identity to SEQ ID NO:2. Furthermore, those with at least 97-99% are highly preferred. Also included within hSLNAC1 polypeptides are polypeptides having the amino acid sequence which have at least 80% identity to the polypeptide having the amino acid sequence of SEQ ID NO:2 over its entire length, and still more preferably at least 90% identity, and even still more preferably at least 95% identity to SEQ ID NO:2. Furthermore, those with at least 97-99% are highly preferred. hSLNAC1 polypeptide comprising an amino acid sequence having at least 80% identity, preferably at least 90% identity, and even still more preferably at least 95% to the amino acid sequence encoded by the cDNA contained in ATCC 97987 are also included within the present invention. Preferably hSLNAC1 polypeptides exhibit at least one biological activity of the receptor.

The hSLNAC1 polypeptides may be in the form of the 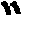 mature*"* protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the hSLNAC1 polypeptides are also included in the invention. A fragment is a polypeptide having an amino acid sequence that entirely is the same as part, but not all, of the amino acid sequence of the aforementioned hSLNAC1 polypeptides. As with hSLNAC1 polypeptides, fragments may be 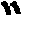 free-standing,*"* or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of hSLNAC1 polypeptide. In this context 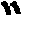 about*"* includes the particularly recited ranges larger or smaller by several, 5, 4, 3, 2 or 1 amino acid at either extreme or at both extremes.

Preferred fragments include, for example, truncation polypeptides having the amino acid sequence of hSLNAC1 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate receptor activity, including those with a similar activity or an improved activity, or with a decreased undesirable activity. Also included are those that are antigenic or immunogenic in an animal, especially in a human.

Preferably, all of these polypeptide fragments retain the biological activity of the receptor, including antigenic activity. Variants of the defined sequence and fragments also form part of the present invention. Preferred variants are those that vary from the referents by conservative amino acid substitutions i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The hSLNAC1 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to hSLNAC1 polynucleotides. hSLNAC1 polynucleotides include isolated polynucleotides which encode the hSLNAC1 polypeptides and fragments, and polynucleotides closely related thereto. More specifically, hSLNAC1 polynucleotide of the invention include a polynucleotide comprising the nucleotide sequence set forth in SEQ ID NO:1 encoding a hSLNAC1 polypeptide of SEQ ID NO : 2, and polynucleotide having the particular sequence of SEQ ID NO:1.

hSLNAC1 polynucleotides further include a polynucleotide comprising a nucleotide sequence that has at least 80% identity to a nucleotide sequence encoding the hSLNAC1 polypeptide of SEQ ID NO : 2 over its entire length, and a polynucleotide that is at least 80% identical to that having SEQ ID NO : 1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under hSLNAC1 polynucleotides are a nucleotide sequence which has sufficient identity to a nucleotide sequence contained in SEQ ID NO:1 or contained in the cDNA insert in the plasmid deposited with the ATCC Deposit number 97987 (see herein-below) to hybridize under conditions useable for amplification or for use as a probe or marker. Moreover, hSLNAC1 polynucleotide include a nucleotide sequence having at least 80% identity to a nucleotide sequence encoding the hSLNAC1 polypeptide expressed by the cDNA insert deposited at the ATCC with Deposit Number 97987, and a nucleotide sequence comprising at least 15 contiguous nucleotides of such cDNA insert. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. The invention also provides polynucleotides which are complementary to all the above hSLNAC1 polynucleotides.

A deposit containing a hSLNAC1 cDNA has been deposited with the American Type Culture Collection (ATCC), 12301 Park Lawn Drive, Rockville, Maryland 20852, USA, on April 16, 1997, and assigned ATCC Deposit Number 97987. The deposited material (clone) is plasmid pcDNA3 which can be obtained from Invitrogen, Inc. containing that further contains the full length hSLNAC1 cDNA, referred to as p3SLNAC1 upon deposit. The cDNA insert is within KpnI-NotI sites in the vector. The nucleotide sequence of the polynucleotides contained in the deposited material, as well as the amino acid sequence of the polypeptide encoded thereby, are controlling in the event of any conflict with any description of sequences herein.

The deposit has been made under the terms of the Budapest Treaty on the international recognition of the deposit of micro-organisms for purposes of patent procedure. The strain will be irrevocably and without restriction or condition released to the public upon request.

hSLNAC1 of the invention is structurally related to other proteins of the sodium channel family, as shown by the results of sequencing the cDNA of SEQ ID NO:1. The cDNA sequence of SEQ ID NO:1 contains an open reading frame (nucleotide number 76 to 1629) encoding a polypeptide of 518 amino acids of SEQ ID NO:2.

Amino acid sequence of SEQ ID NO:2 has about 52,1% identity and 71,8% similarity(using GCG gap algorythm) in 518 amino acid residues with ASIC Protein (gi/2039366) Waldmann R. *et al., Nature*, (1997) 386:173-177}. Nucleotide sequence of SEQ ID NO:1 has about 60,7% identity (using GCG gap algorythm) in 1554 nucleotide residues with ASIC Protein (gi/2039365).

One polynucleotide of the present invention encoding hSLNAC1 may be obtained using standard cloning and screening, from a cDNA library derived from mRNA in cells of human cerebellum using the expressed sequence tag (EST) analysis (Adams, M.D., *et al. Science* (1991) 252:1651-1656; Adams, M.D. *et al., Nature*, (1992) *355*:632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding hSLNAC1 polypeptide of SEQ ID NO:2 may be identical to the polypeptide encoding sequence contained in SEQ ID NO:1 (nucleotide number 76 to 1629), or it may be a sequence, which as a result of the redundancy (degeneracy) of the genetic code, also encodes the polypeptide of SEQ ID NO:2.

When the polynucleotides of the invention are used for the recombinant production of hSLNAC1 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding hSLNAC1 variants comprising the amino acid sequence of hSLNAC1 polypeptide of SEQ ID NO : 2 in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term 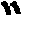 stringent conditions*"* means hybridization will occur only if there is at least 95% and preferably at least 97% identity between the sequences.

Polynucleotides of the invention, which are identical or sufficiently identical to a nucleotide sequence contained in Figure 1 or a fragment thereof, or to the cDNA insert in the plasmid deposited at the ATCC with Deposit Number 97987 or a fragment thereof, may be used as hybridization probes for cDNA and genomic DNA, to isolate full-length cDNAs and genomic clones encoding hSLNAC1 and to isolate cDNA and genomic clones of other genes that have a high sequence similarity to the hSLNAC1 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding hSLNAC1 polypeptide comprises the steps of screening an appropriate library under stingent hybridization conditions with a labeled probe having the SEQ ID NO : 1 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors and Host Cells

The present invention also relates to vectors which include the isolated DNA molecules of the present invention, host cells which are genetically engineered with the recombinant vectors, and the production of hSLNAC1 polypeptides or fragments thereof by recombinant techniques.
The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged *in vitro* using an appropriate packaging cell line and then transduced into host cells.

The DNA insert should be operatively linked to an appropriate promoter, such as the phage lambda PL promoter, the *E. coli lac, trp* and *tac* promoters, the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the mature transcripts expressed by the constructs will preferably include a translation initiating at the beginning and a termination codon (UAA, UGA or UAG) appropriately positioned at the end of the polypeptide to be translated.

As indicated, the expression vectors will preferably include at least one selectable marker. Such markers include dihydrofolate reductase , zeocin, hygromycin, or neomycin resistance for eukaryotic cell culture and tetracycline or ampicillin resistance genes for culturing in *E. coli* and other bacteria. Representative examples of appropriate heterologous hosts include, but are not limited to, bacterial cells, such as *E. coli*, *Streptomyces* and *Salmonella typhimurium* cells; fungal cells, such as yeast cells; insect cells such as *Drosophila* S2 and *Spodoptera* Sf9 cells; animal cells such as CHO, COS and Bowes melanoma cells; and plant cells. Appropriate culture mediums and conditions for the above-described host cells are known in the art.

Among vectors preferred for use in bacteria include pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals, such as Davis *et al., Basic Methods In Molecular Biology* (1986).

The polypeptide may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP-A-O 464 533 (Canadian counterpart 2045869) discloses fusion proteins comprising various portions of constant region of immunoglobulin molecules together with another human protein or part thereof. In many cases, the Fc part in a fusion protein is thoroughly advantageous for use in therapy and diagnosis and thus results, for example, in improved pharmacokinetic properties (EP-A 0232 262). On the other hand, for some uses it would be desirable to be able to delete the Fc part after the fusion protein has been expressed, detected and purified in the advantageous manner described. This is the case when Fc portion proves to be a hindrance to use in therapy and diagnosis, for example when the fusion protein is to be used as antigen for immunizations. In drug discovery, for example, human proteins, such as, hIL5- has been fused with Fc portions for the purpose of high-throughput screening assays to identify antagonists of hIL-5. See, D. Bennett *et al., Journal of Molecular Recognition, Vol*. 8:52-58 (1995) and K. Johanson *et al., The Journal of Biological Chemistry, Vol*. *270*, No. 16:9459-9471 (1995).

The hSLNAC1 receptor can be recovered and purified from recombinant cell cultures by well-known methods, including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Polypeptides of the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes.

### Diagnostic Assays

This invention also relates to the use of hSLNAC1 polynucleotides for use as diagnostic reagents. Detection of a mutated form of hSLNAC1 gene associated with a dysfunction will provide a diagnostic tool that can add to or define a diagnosis of a disease or susceptibility to a disease which results from under-expression, over-expression or altered expression of hSLNAC1. Individuals carrying mutations in the hSLNAC1 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled hSLNAC1 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising hSLNAC1 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The diagnostic assays offer a process for diagnosing or determining a susceptibility to the treatment of diseases through detection of mutation in the hSLNAC1 gene by the methods described. Said diseases are, in particular, chosen among the following : neuronal degenerescence problems, hyperalgesia, Alzeihmer disease, Parkinson disease, chorea, muscular spasm, epilepsy, stroke, cardiac diseases, schizophrenia, depression, nicotine dependence, morphine dependence, amyotrophic lateral sclerosis, multiple sclerosis, inflammation, pain, cancer, obesity, to mimic or antagonize effect of some endogenous transmitter peptides in the central or peripheral nervous system (such as for instance opioïds or anti-opioïds), alteration of gustative perception, disorder related to abnormal expression of the hSLNAC1 protein.

In addition, the same diseases can be diagnosed by methods comprising determining from a sample derived from a subject an abnormally decreased or increased level of hSLNAC1 polypeptide or hSLNAC1 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an hSLNAC1, in a sample derived from a host are well-known to those of skill in the art. Such assay methods include radioimmunoassays, competitive-binding assays, Western Blot analysis and ELISA assays.

### Detection of hSLNAC1 Gene Expression

The expression level of the *hSLNAC1* gene can be readily assayed by one of ordinary skill in the art. By "assaying the expression level of the gene encoding the hSLNAC1 polypeptide" is intended qualitatively or quantitatively measuring or estimating the level of the hSLNAC1 polypeptide or the level of the mRNA encoding the hSLNAC1 polypeptide in a biological sample (e.g., by determining or estimating absolute protein level or mRNA level).
By "biological sample" is intended any biological sample obtained from an individual, cell line, tissue culture, or other source which contains hSLNAC1 polypeptide or hSLNAC1 mRNA. Such tissues include cerebellum, brain, midbrain, spinal cord, nerve endings, retina, breast, pituitary, heart, placenta, lung, skeletal muscle, kidney, and pancreas. Biological samples include mammalian tissues which contain hSLNAC1 polypeptide. Preferred mammals include monkeys, apes, cats, dogs, cows, pigs, horses, rabbits, and humans. Particularly preferred are humans.
Total cellular RNA can be isolated from a biological sample using the single-step guanidinium-thiocyanate-phenolchloroform method described in Chomczynski and Sacchi (*Anal. Biochem*. *162*:156-159 (1987)). Levels of mRNA encoding the hSLNAC1 receptor are then assayed using any appropriate method. These include Northern blot analysis (Harada *et al., Cell 63*:303-312 (1990)), S1 nuclease mapping (Harada *et al., Cell 63*:303-312 (1990)), the polymerase chain reaction (PCR), reverse transcription in combination with the polymerase chain reaction (RT-PCR) (Fujita *et al., Cell 49*:35-36 (1990)), and reverse transcription in combination with the ligase chain reaction (RT-LCR).

As discussed here-above, assaying hSLNAC1 polypeptide levels in a biological sample can occur using antibody-based techniques. For example, hSLNAC1 polypeptide expression in tissues can be studied with classical immunohistological methods (Jalkanen, M. *et al., J. Cell. Biol. 101*:976-985 (1985); Jalkanen, M. *et al., J. Cell. Biol. 105*: 3087-3096 (1987)). Other antibody-based methods useful for detecting hSLNAC1 polypeptide gene expression include immunoassays, such as the enzyme linked immunosorbent assay (ELISA) and the radioimmunoassay (RIA). Suitable labels are known in the art and include enzyme labels, such as glucose oxidase, and radioisotopes, such as iodine (¹²⁵I, ¹²¹I), carbon (¹⁴C), sulfur (³⁵S), tritium (³H), indium (¹¹²In), and technetium (^{99m}Tc), biotin and fluorescent labels, such as fluorescein and rhodamine.

### Chromosome Assays

The nucleic acid molecules of the present invention are also valuable for chromosome identification. The sequence is specifically targeted to and can hybridize with a particular location on an individual human chromosome. The mapping of DNAs to chromosomes according to the present invention is an important first step in correlating those sequences with genes associated with disease.
In certain preferred embodiments in this regard, the cDNA herein disclosed is used to clone genomic DNA of a hSLNAC1 polypeptide gene. This can be accomplished using a variety of well known techniques and libraries, which generally are available commercially. The genomic DNA then is used for *in situ* chromosome mapping using well known techniques for this purpose.

In addition, in some cases, sequences can be mapped to chromosomes by preparing PCR primers (preferably 15-25 bp) from the cDNA. Computer analysis of the 3' untranslated region of the gene is used to rapidly select primers that do not span more than one exon in the genomic DNA, thus complicating the amplification process. These primers are then used for PCR screening of somatic cell hybrids containing individual human chromosomes.
Fluorescence in situ hybridization ("FISH") of a cDNA clone to a metaphase chromosomal spread can be used to provide a precise chromosomal location in one step. This technique can be used with probes from the cDNA as short as 50 or 60 bp. For a review of this technique, see Verma *et al., Human Chromosomes: A Manual Of Basic Techniques*, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance In Man, available on-line through Johns Hopkins University, Welch Medical Library. The relationship between genes and diseases that have been mapped to the same chromosomal region are then identified through linkage analysis (coinheritance of physically adjacent genes). Next, it is necessary to determine the differences in the cDNA or genomic sequence between affected and unaffected individuals. If a mutation is observed in some or all of the affected individuals but not in any normal individuals, then the mutation is likely to be the causative agent of the disease.
Having generally described the invention, the same will be more readily understood by reference to the following examples, which are provided by way of illustration and are not intended as limiting.

### Antibodies

The polypeptides of the invention or their fragments or analogs thereof, or cells expressing them can also be used as immunogens to produce antibodies immunospecific for the hSLNAC1 polypeptides. The term 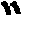 immunospecific*"* means that the antibodies have substantiall greater affinity for the polypeptides of the invention than their affinity for other related polypeptides in the prior art.

Antibodies generated against the hSLNAC1 polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., Nature (1975) 256:495-497), the trioma technique, the human B-cell hybridoma technique (Kozbor et al., Immunology Today (1983) 4:72) and the EBV-hybridoma technique (Cole et al., MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.
Non-limiting examples of polypeptides or peptides that can be used to generate hSLNAC1 polypeptide-specific antibodies include: a polypeptide comprising amino acid residues from about 50 to about 56 of SEQ ID NO 2 ; a polypeptide comprising amino acid residues from about 71 to about 81 of SEQ ID NO 2; a polypeptide comprising amino acid residues from about 105 to about 112 of SEQ ID NO 2; a polypeptide comprising amino acid residues from about 190 to about 209 of SEQ ID NO 2 ; a polypeptide comprising amino acid residues from about 215 to about 225 of SEQ ID NO 2; a polypeptide comprising amino acid residues from about 262 to about 272 of SEQ ID NO 2; a polypeptide comprising amino acid residues from about 277 to about 285 of SEQ ID NO 2; a polypeptide comprising amino acid residues from about 316 to about 327 of SEQ ID NO 2; a polypeptide comprising amino acid residues from about 369 to about 380 of SEQ ID NO 2; a polypeptide comprising amino acid residues from about 394 to about 405 of SEQ ID NO 2; a polypeptide comprising amino acid residues from about 446 to about 464 of SEQ ID NO 2; a polypeptide comprising amino acid residues from about 492 to about 500 of SEQ ID NO 2; and a polypeptide comprising amino acid residues from about 510 to about of SEQ ID NO 2. As indicated above, the inventors have determined that the above polypeptide fragments are antigenic regions of the hSLNAC1 polypeptide.

The above-mentioned antibodies may be employed to isolate or to identify clones expressing the polypeptide or to purify the polypeptides by affinity chromatography.
Antibodies against hSLNAC1 polypeptides may also be employed to treat, among others, patients with nervous system disorders, to treat neuronal degenerescence problems,Alzeihmer disease, Parkinson disease, chorea, muscular spasm, epilepsy, stroke, cardiac diseases, schizophrenia, depression, nicotine dependence, morphine dependence, amyotrophic lateral sclerosis, inflammation, pain, multiple sclerosis, cancer, obesity, to mimic or antagonize effect of some endogenous transmitter peptides in the central or peripheral nervous system (such as for instance opioïds or anti-opioïds), to alter gustative perception, to cause analgesia or anesthesia, or to treat any disorder related to abnormal expression of said hSLNAC1 polypeptide.

### Vaccines

Another aspect of the invention relates to a method for inducing an immunological response in a mammal which comprises inoculating the mammal with hSLNAC1 polypeptide, or a fragment thereof, adequate to produce antibody and/or T cell immune response to protect said animal from , among others, nervous system disorders, neuronal degenerescence problems,Alzeihmer disease, Parkinson disease, chorea, muscular spasm, epilepsy, stroke, cardiac diseases, schizophrenia, depression, nicotine dependence, morphine dependence, amyotrophic lateral sclerosis, inflammation, pain, multiple sclerosis, cancer, obesity, to mimic or antagonize effect of some endogenous transmitter peptides in the central or peripheral nervous system (such as for instance opioïds or anti-opioïds), to cause analgesia or anesthesia, or to treat any disorder related to abnormal expression said hSLNAC1 polypeptide.
Yet another aspect of the invention relates to a method of inducing immunological response in a mammal which comprises, delivering hSLNAC1 polypeptide via a vector directing expression of hSLNAC1 polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a hSLNAC1 polypeptide wherein the composition comprises a hSLNAC1 polypeptide or hSLNAC1 gene. The vaccine formulation may further comprise a suitable carrier. Since hSLNAC1 polypeptide may be broken down in the stomach, it is preferably administered parenterally (including subcutaneous, intramuscular, intravenous or intradermal injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The hSLNAC1 polypeptide of the present invention may be employed in a screening process for compounds which bind the receptor and which activate (agonists) or inhibit activation of (antagonists) the receptor polypeptide of the present invention. Thus, polypeptides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics. See Coligan *et al., Current Protocols in Immunology* 1(2) Chapter 5 (1991).

hSLNAC1 polypeptides may be responsible for many biological functions, including many pathologies. Accordingly, it is desirous to find compounds and drugs which stimulate hSLNAC1 on the one hand and which can inhibit the function of hSLNAC1 on the other hand. In general, agonists are employed for therapeutic and prophylactic purposes for such conditions as, among others, nervous system disorders, to treat neuronal degenerescence problems,Alzeihmer disease, Parkinson disease, chorea, muscular spasm, epilepsy, stroke, cardiac diseases, schizophrenia, depression, nicotine dependence, morphine dependence, amyotrophic lateral sclerosis, inflammation, pain, multiple sclerosis, cancer, obesity, to mimic or antagonize effect of some endogenous transmitter peptides in the central or peripheral nervous system (such as for instance opioïds or anti-opioïds), to alter gustative perception, to cause analgesia or anesthesia, or to treat any disorder related to abnormal expression of said hSLNAC1 polypeptide.

Antagonists may be employed for a variety of therapeutic and prophylactic purposes for such conditions as, among others, nervous system disorders, to treat neuronal degenerescence problems,Alzeihmer disease, Parkinson disease, chorea, muscular spasm, epilepsy, stroke, cardiac diseases, schizophrenia, depression, nicotine dependence, morphine dependence, amyotrophic lateral sclerosis, inflammation, pain, multiple sclerosis, cancer, obesity, to mimic or antagonize effect of some endogenous transmitter peptides in the central or peripheral nervous system (such as for instance opioïds or anti-opioïds), to alter gustative perception, to cause analgesia or anesthesia, or to treat any disorder related to abnormal expression of said hSLNAC1 polypeptide.

In general, such screening procedures involve producing appropriate cells which express the receptor polypeptide of the present invention on the surface thereof. Such cells include cells from mammals, yeast, *Drosophila or E. coli.* Cells expressing the receptor (or cell membrane containing the expressed receptor) are then contacted with a test compound to observe binding, or stimulation or inhibition of a functional response.

The assays may simply test binding of a candidate compound wherein adherence to the cells bearing the receptor is detected by means of a label directly or indirectly associated with the candidate compound or in an assay involving competition with a labeled competitor. Further, these assays may test whether the candidate compound results in a signal generated by activation of the receptor, using detection systems appropriate to the cells bearing the receptor at their surfaces. Inhibitors of activation are generally assayed in the presence of a known agonist and the effect on activation by the agonist by the presence of the candidate compound is observed. Standard methods for conducting such screening assays are well understood in the art.

Alternatively, it is also possible to mutate the hSLNAC1 cDNA in order to produce a constitutively active sodium channel (Waldmann et al., J. Biol. Chem.271 pp 10433-10436 ; Huang and Chalfie, Nature 367, 467-470). The mutation may for instance consists in changing glycine 411 to phenylalanine. Then, the constitutively active sodium channel may be expressed in host cells to produce a screening assay where sodium channel activity is permanent. The recording of channel activity may be carried out either by membrane voltage analysis, directly (patch clamp for example) or indirectly (fluorescent probes for example), or by sodium entry measurement (radioactive sodium influx, fluorescent probes or reporter genes for example).

Examples of potential hSLNAC1 antagonists include antibodies or, in some cases, oligonucleotides or proteins which are closely related to the ligand of the hSLNAC1, e.g., a fragment of the ligand, or small molecules which bind to the receptor but do not elicit a response, so that the activity of the receptor is prevented.

### Prophylactic and Therapeutic Methods

This invention provides methods of treating an abnormal conditions related to both an excess of and insufficient amounts of hSLNAC1 activity.

If the activity of hSLNAC1 is in excess, several approaches are available. One approach comprises administering to a subject an inhibitor compound (antagonist) as hereinabove described along with a pharmaceutically acceptable carrier in an amount effective to inhibit activation by blocking binding of ligands to the hSLNAC1, or by inhibiting a second signal, and thereby alleviating the abnormal condition.

In another approach, soluble forms of hSLNAC1 polypeptides still capable of binding the ligand in competition with endogenous hSLNAC1 may be administered. Typical embodiments of such competitors comprise fragments of the hSLNAC1 polypeptide.

In still another approach, expression of the gene encoding endogenous hSLNAC1 can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo*.

For treating abnormal conditions related to an under-expression of hSLNAC1 and its activity, several approaches are also available. One approach comprises administering to a subject a therapeutically effective amount of a compound which activates hSLNAC1, i.e., an agonist as described above, in combination with a pharmaceutically acceptable carrier, to thereby alleviate the abnormal condition. Alternatively, gene therapy may be employed to effect the endogenous production of hSLNAC1 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo*. For overview of gene therapy, see Chapter 20, *Gene Therapy and other Molecular Genetic-based Therapeutic Approaches*, (and references cited therein) in Human Molecular Genetics, T Strachan and A P Read, BIOS Scientific Publishers Ltd (1996).

### Formulation and Administration

Peptides, such as the soluble form of hSLNAC1 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as 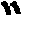 gene therapy*"* as described above. Thus, for example, cells from a subject may be engineered with a polynucleotide, such as a DNA or RNA, to encode a polypeptide *ex vivo*, and for example, by the use of a retroviral plasmid vector. The cells are then introduced into the subject.

### Examples

The examples below are carried out using standard techniques, which are well known and routine to those of skill in the art, except where otherwise described in detail. The examples illustrate, but do not limit the invention.

### Example 1 :

### Cloning the Human Sodium Channel

The sequence of the hSLNAC1 was first identified by searching a database containing approximately 1 million human ESTs, which was generated using high throughput automated DNA sequence analysis of randomly selected human cDNA clones (Adams, M.D. *et al., Nature* 377:3-174 (1995); Adams, M.D. *et al., Nature* 355:632-634 (1992); and Adams, M.D. *et al., Science 252*:1651-1656 (1991)). Sequence homology comparisons of each EST were performed against the GenBank database using the blastn and tblastn algorithms (Altschul, S.F. *et al., J. Mol. Biol. 215*:403-410 (1990)). A specific homology search using the known human MDEG amino acid sequence against this human EST database revealed one EST (HGS826465), from a cerebellum cDNA library, with approximatively 50% similarity to MDEG. HGS826465 contains 1710 bp and the sequence comparison suggested that it contained the complete open reading frame of a new protein. Sequence of the gene was confirmed by double strand DNA sequencing using the TaqFs (Perkin Elmer) and the gene was shown to be completely new by a blast search against Genbank release 98. An expression vector construct was made by inserting the KpnI-NotI fragment carrying the entire hSLNAC1 coding region into the KpnI-NotI site of the expression vector pcDNA3(-) (Invitrogen, Inc). This construct was named p3SLNAC1.

### Example 2

### Cloning and Expression of hSLNAC1 in Mammalian Cells

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as PSVL and PMSG (Pharmacia, Uppsala, Sweden), pRSVcat (ATCC 37152), pSV2dhfr (ATCC 37146) and pBC12MI (ATCC 67109) and pcDNA3(-) (Invitrogen). Mammalian host cells that could be used include, human Hela 293, H9 and Jurkat cells, mouse NIH3T3 and C127 cells, Cos 1, Cos 7 and CV 1, quail QC1-3 cells, mouse L cells and Chinese hamster ovary (CHO) cells.
Alternatively, the gene can be expressed in stable cell lines that contain the gene integrated into a chromosome. The co-transfection with a selectable marker such as dhfr, gpt, neomycin, zeocin or hygromycin allows the identification and isolation of the transfected cells. The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy *et al., Biochem. J. 227*:277-279 (1991); Bebbington *et al., Bio/Technology 10*:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.
The expression vector pcDNA3(-) contains the strong promoter (CMV) of the Cytomegalovirus. Multiple cloning sites, e.g., with the restriction enzyme cleavage sites KpnI, NotI, facilitate the cloning of the gene of interest. The vectors contain in addition the 3' intron, the polyadenylation and termination signal of the bovine growth hormone gene.

### Example 2(a): Cloning and Expression in COS Cells

The expression plasmid, p3SLNAC1, is made by cloning a cDNA encoding *hSLNAC1* into the expression vector pcDNA3(-) (which can be obtained from Invitrogen, Inc.). The expression vector pcDNA3(-) contains: (1) an *E. coli* origin of replication effective for propagation in *E. coli* and other prokaryotic cells; (2) an ampicillin resistance gene for selection of plasmid-containing prokaryotic cells; (3) an SV40 origin of replication for propagation in eukaryotic cells; (4) a CMV promoter, a polylinker ; (5) a polyadenylation from the bovine growth hormone gene arranged so that a cDNA can be conveniently placed under expression control of the CMV promoter and operably linked to the polyadenylation signal by means of restriction sites in the polylinker. pcDNA3(-) contains, in addition, the selectable neomycin marker.

A DNA fragment encoding the hSLNAC1 polypeptide is cloned into the polylinker region of the vector so that recombinant protein expression is directed by the CMV promoter. The plasmid construction strategy is as follows. The *hSLNAC1* cDNA of the deposited clone is in the bluescript vector (Stratagene), it is excised from the bluescript vector with KpnI and Not I. The vector, pcDNA3(-), is digested with KpnI and Not I. The PCR amplified DNA fragment and the linearized vector are then ligated. The ligation mixture is transformed into *E. coli* strain DH5α (available from GIBCO BRL), and the transformed culture is plated on ampicillin media plates which then are incubated to allow growth of ampicillin resistant colonies. Plasmid DNA is isolated from resistant colonies and examined by restriction analysis or other means for the presence of the hSLNAC1-encoding fragment.

For expression of recombinant hSLNAC1 polypeptide, COS cells are transfected with an expression vector, as described above, using DEAE-Dextran, as described, for instance, in Sambrook *et al., Molecular Cloning: a Laboratory Manual*, Cold Spring Laboratory Press, Cold Spring Harbor, New York (1989). Cells are incubated under conditions for expression of hSLNAC1 by the vector.

### Example 2(b): Cloning and Expression in HEK293 Cells

The vector p3SLNAC1 is used for the expression of hSLNAC1 protein. Plasmid p3SLNAC1 is described in example 1. The plasmid contains the mouse neomycin resistance gene under control of the SV40 early promoter. HEK293 Cells are transfected with these plasmids can be selected by growing the cells in a selective medium (containing 1 mg/ml Geneticin, Life Technologies). Cells grown in presence of 1mg/ml concentrations of Geneticin develop resistance to the drug by producing the target enzyme, Neomycin Resistance. If a second gene is linked to the Neomycin Resistance gene, it is usually co-expressed. It is known in the art that this approach may be used to develop cell lines expressing large amounts of the protein of interest, up to 1 pmole per mg of cell membrane in the case of a receptor. Subsequently, when the Geneticin is withdrawn, cell lines are obtained which contain the amplified gene integrated into one or more chromosome(s) of the host cell.
Clontech's Tet-Off and Tet-On gene expression systems and similar systems can be used to express hSLNAC1 in a regulated way in mammalian cells (Gossen, M. and Bujard, H., *Proc. Natl. Acad. Sci. USA 89*: 5547-5551(1992)). For the polyadenylation of the mRNA other signals, e.g., from the human growth hormone or globin genes can be used as well. Stable cell lines carrying a gene of interest integrated into the chromosomes can also be selected upon co-transfection with a selectable marker such as gpt, G418 or hygromycin.

HEK293 cells are used for transfection. 20 µg of the expression plasmid p3SLNAC1 is transfected using calcium phosphate (Chen C, Okayama H, (1987) Mol. Cell. Biol. ; 7 : 2745-2752.). The plasmid pcDNA3(-) contains a dominant selectable marker, the neomycin resistance gene from Tn5 encoding an enzyme that confers resistance to a group of antibiotics including Geneticin. The cells are seeded in MEM supplemented with 1 mg/ml Geneticin. After 2 days, the cells are trypsinized and seeded in cloning plates in MEM supplemented with 1 mg/ml Geneticin. After about 10-14 days, single clones are trypsinized and then seeded in 6-well petri dishes or 10 ml flasks. Clones growing are then transferred to new 6-well plates. Expression of the desired gene product is analyzed, for instance, by Northern blot.

### Example 3

### Tissue distribution of hSLNAC1 mRNA expression

Northern blot analysis can be carried out to examine hSLNAC1 gene expression in human tissues, using methods described by, among others, Sambrook *et al*., cited above. A cDNA probe containing the entire nucleotide sequence of the hSLNAC1 protein (SEQ ID NO : 1) can be labeled with ³²P using the Rediprime™ DNA labeling system (Amersham Life Science, Arlington, IL), according to manufacturer's instructions. After labeling, the probe can be purified using a CHROMA SPIN- 100™ column (Clontech Laboratories, Inc.), according to manufacturer's protocol number PT1200-1. The purified labeled probe was then used to examine various human tissues for *hSLNAC1* mRNA.

Multiple Tissue Northern (MTN) blots containing various human tissues can be obtained from Clontech and examined with the labeled probe using ExpressHyb™ hybridization solution (Clontech) according to manufacturer's protocol number PT1190-1. Following hybridization and washing, the blots can be mounted and exposed to film at -70°C overnight, and films developed according to standard procedures. It will be clear that the invention may be practiced otherwise than as particularly described in the foregoing description and examples.

Numerous modifications and variations of the present invention are possible in light of the above teachings and, therefore, are within the scope of the appended claims. The entire disclosure of all publications (including patents, patent applications, journal articles, laboratory manuals, books, or other documents) cited herein are incorporated by reference.

## Claims

1. An isolated polynucleotide selected from the group consisting of :
(a) a polynucleotide comprising a nucleotide sequence that has at least 80% identity to a nucleotide sequence encoding the hSLNAC1 polypeptide of SEQ ID NO:2 over its entire length; or a nucleotide sequence complementary to said nucleotide sequence,
(b) a polynucleotide comprising a nucleotide sequence that has at least 80% identity to a nucleotide sequence encoding the hSLNAC1 polypeptide expressed by the cDNA insert deposited at the ATCC with Deposit Number 97987; or a nucleotide sequence complementary to said nucleotide sequence.

2. A polynucleotide according to claim 1 which is DNA or RNA.

3. A polynucleotide according to one of claim 1 and 2 wherein said nucleotide sequence is at least 80% identical to that contained in SEQ ID NO:1.

4. A polynucleotide according to claim 3 wherein said nucleotide sequence comprises the hSLNAC1 polypeptide encoding sequence contained in SEQ ID NO:1.

5. A polynucleotide according to claim 3 which is polynucleotide of SEQ ID NO: 1.

6. A DNA or RNA molecule comprising an expression system, wherein said expression system is capable of producing a hSLNAC1 polypeptide comprising an amino acid sequence, which has at least 80% identity with the polypeptide of SEQ ID NO:2 when said expression system is present in a compatible host cell.

7. A host cell comprising the expression system of claim 6.

8. A process for producing a hSLNAC1 polypeptide comprising culturing a host of claim 7 under conditions sufficient for the production of said polypeptide and recovering the polypeptide from the culture.

9. A process for producing a cell which produces a hSLNAC1 polypeptide thereof comprising transforming or transfecting a host cell with the expression system of claim 6 such that the host cell, under appropriate culture conditions, produces a hSLNAC1 polypeptide.

10. A hSLNAC1 polypeptide comprising an amino acid sequence which is at least 80% identical to the amino acid sequence of SEQ ID NO:2 over its entire length.

11. The polypeptide of claim 10 which comprises the amino acid sequence of SEQ ID NO:2.

12. A hSLNAC1 polypeptide comprising an amino acid sequence which is at least 80% identical to the amino acid sequence encoded by the cDNA contained in ATCC 97987.

13. An antibody immunospecific for the hSLNAC1 polypeptide of one of claims 10 to 12.

14. A method for the treatment of a subject in need of enhanced activity or expression of hSLNAC1 polypeptide of one of claims 10 to 13 comprising:
(a) administering to the subject a therapeutically effective amount of an agonist to said hSLNAC1 polypeptide; and/or
(b) providing to the subject polynucleotide of claim 1 in a form so as to effect production of said hSLNAC1 polypeptide activity *in vivo*.

15. A method for the treatment of a subject having need to inhibit activity or expression of hSLNAC1 polypeptide of one of claims 10 to 12 comprising:
(a) administering to the subject a therapeutically effective amount of an antagonist to said hSLNAC1 polypeptide; and/or
(b) administering to the subject a nucleic acid molecule that inhibits the expression of the nucleotide sequence encoding said hSLNAC1 polypeptide; and/or
(c) administering to the subject a therapeutically effective amount of a polypeptide that competes with said hSLNAC1 polypeptide for its ligand.

16. A process for diagnosing a disease or a susceptibility to a disease in a subject related to expression or activity of hSLNAC1 polypeptide of one of claims 10 to 12 in a subject comprising:
(a) determining the presence or absence of a mutation in the nucleotide sequence encoding said hSLNAC1 polypeptide in the genome of said subject; and/or
(b) analyzing for the presence or amount of the hSLNAC1 polypeptide expression in a sample derived from said subject.

17. A method for identifying agonists to hSLNAC1 polypeptide of one of claims 10 to 12 comprising:
(a) contacting cells produced by claim 9 with a candidate compound; and
(b) determining whether the candidate compound effects a signal generated by activation of the hSLNAC1 polypeptide.

18. An agonist identified by the method of claim 17.

19. The method for identifying antagonists to hSLNAC1 polypeptide of one of claims 10 to 12 comprising:
(a) contacting said cell produced by claim 9 with an agonist; and
(b) determining whether the signal generated by said agonist is diminished in the presence of a candidate compound.

20. An antagonist identified by the method of claim 19.
